# EUROPEAN PATENT SPECIFICATION

(11) **EP 1 049 466 B1**
(45) Date of publication and mention of the grant of the patent: **07.12.2005**
(21) Application number: 99904804.4
(22) Date of filing: 21.01.1999
(51) Int. Cl.: A61K 31/41, A61K 31/38

(54) **CARBONIC ANHYDRASE INHIBITOR FOR INCREASING OXYGEN TENSION IN THE OPTIC NERVE AND RETINA**
CARBONISCHE ANHYDRASE INHIBITOR ZUR STEIGERUNG DES SAUERSTOFFDRUCKS IM SEHNERV UND RETINA
TECHNIQUE VISANT A ACCROITRE LA TENSION EN OXYGENE DU NERF OPTIQUE ET DE LA RETINE

(30) Priority: 23.01.1998 US 72360 P; 07.05.1998 GB 9809787
(43) Date of publication of application: 08.11.2000
(73) Proprietor: Stefansson, Einar, 110 Reykjavik (IS)
(72) Inventor: STEFANSSON, Einar, IS-110 Reykjavik (IS); DOLLERUP, Jens, DK-2600 Glostrup (DK); BANG, Kurt, DK-2600 Glostrup (DK)
(74) Representative: Fridriksson, Einar Karl
(86) International application number: PCT/EP1999/000409
(87) International publication number: WO 1999/037292

(56) References cited:
- EP-A- 0 286 903
- EP-A- 0 509 753
- WO-A-93/16701
- WO-A-96/37203
- EYSTEINSSON ET AL: "the effect of carbonic anhydrase inhibitors on optic nerve oxygen tension in the pig" IOVS, vol. 39, no. 4, 15 March 1998, page 693 XP002106334
- ANDERSON B.: "Ocular effects of changes in oxygen and carbon dioxide tension" TRANS AM OPHTALMOL SOC, vol. 66, 1968, pages 423-474, XP002106335

## Description

### BACKGROUND OF THE INVENTION

Glaucoma is characterized by progressive atrophy of the optic nerve and is frequently associated with elevated intraocular pressure (IOP). The treatment of glaucoma has focused on the elevated lOP and only recently has the attention been shifted more to the optic nerve, where the disease process is predominantly seen. Effective treatment of glaucoma must somehow affect the conditions and metabolism of the optic nerve to stop the atrophic process.

Due to the inaccessibility of the optic nerve, its metabolism has not been thoroughly studied (Novak and Stefansson et al., Exp. Eye Res. 50, 289-96, 1990; and Clin. Exp. Ophthalmol 228, 128-33, 1990). A few reports exist on the blood flow in the optic nerve and retina and how this is affected by glaucoma drugs, such as carbonic anhydrase inhibitors. WO 9316701 discloses treatment of glaucoma with carbonic anhydrase inhibitors (CAIs) such as dorzolamine possibly coupled with a beta-blocker like timolol maleate. EP 286903 discloses compositions comprising a CAI, a beta-blocker and prostaglandin for treating glaucoma. Vorstrup and associates (Vorstrup et al., J. Clin. Invest. 74: 1634-39 (1984)) found that intravenous administration of acetazolamide increased cerebral blood flow. The flow velocity in the intracranial arteries increased whereas a decrease was observed in the ophthalmic artery together with a decrease in ocular pulsatile blood flow (Kerty, et at, Acta Ophthalmol 73: 66-71 (1995) and Acta Ophthalmol 72: 401-8 (1994)), mainly originating from the choroidal circulation. In contrast, Rassam and associates (Rassam et al., Eye 7: 697-702 (1993)) found that intravenous acetazolamide increased retinal blood flow, whereas Grunwald et al., Invest Ophthalmol. Vis Sci 33: 504-7 (1992) found no effect by oral acetazolamide on macular blood flow evaluated with the blue field entopic technique; Harris and associates (Harris et al., Acta Ophthalmol 74: 569-72 (1996)) reported that the application of one drop of dorzolamide 3% decreased the artterio-venous transit time and increased the velocity of fluorescent particles in the paramacular and peripapillary microcirculations, whereas no effect was seen on flow velocity in the retrobulbar vessels.

Ischemia of the microcirculation around the papilla is assumed to be relevant for the pathogenesis of glaucomatous damage to the nerve fiber layer. Several drugs have been tested for their influence on perfusion of the posterior pole of the eye.

The present invention relates to the use of carbonic anhydrase inhibitors to increase vitreal oxygen tension over the optic nerve notwithstanding that oxygen is a powerful retinal vasoconstrictor of the retinal vessels (Hickman & Frayser, Circulation 33, 302-316 (1966)).

### DESCRIPTION OF THE DRAWINGS

Figure 1. Optic nerve oxygen tension reading over time. Time from beginning of record is shown in seconds on the horizontal axis and oxygen tension as mmHg on the vertical axis. At the arrow the breathing mixture is change from 100% O₂ to air (21% O₂).
Figure 2. Optic nerve oxygen tension reading over time. Time from beginning of record is shown in seconds on the horizontal axis and oxygen tension as mmHg on the vertical axis. The middle arrow indicates when the breathing mixture was switched to 5.19% CO₂ and 19.9% O₂. The arrow on the right indicates when the breathing mixture was returned to 20% oxygen (N₂ to balance).
Figure 3. Optic nerve oxygen tension reading over time. Time from beginning of record is shown in seconds on the horizontal axis and oxygen tension as mmHg on the vertical axis. At the arrow 500 mg of dorzolamide was given.
Figure 4. The effects of intravenous dorzolamide on optic nerve oxygen tension. The figure shows six independent recordings where different amounts of dorzolamide were given intravenously at the times indicated by the arrows. Time is shown (in seconds) on the horizontal axis and oxygen tension (mmHg) on the vertical axis.
Figure 5. Optic nerve oxygen tension reading over time. Time from beginning of record is shown in minutes (and hours) on the horizontal axis and oxygen tension as percent atmosphere on the vertical axis. One minute befores mark 9 seven drops of TRUSOPT® (dorzolamide 2%) is applied to the eye.

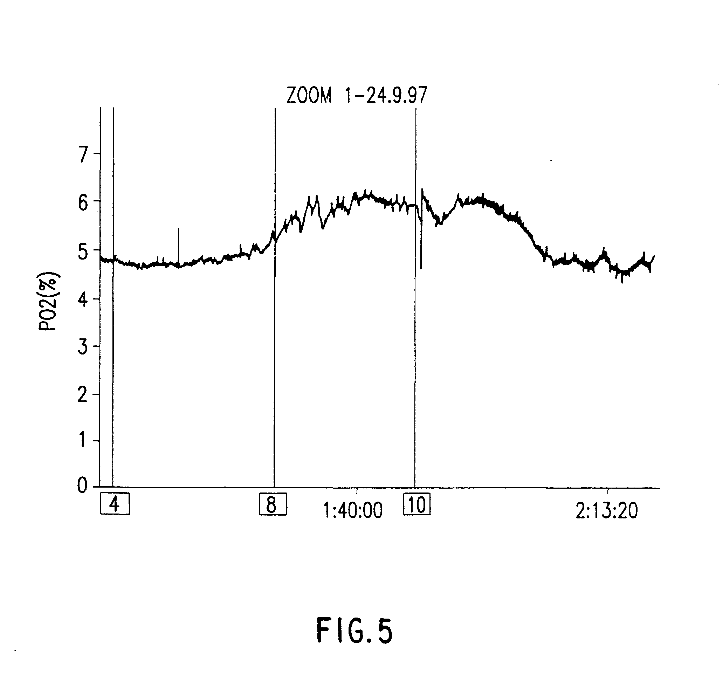

Figure 5. Optic nerve oxygen tension reading over time. Time from beginning of record is shown in minutes (and hours) on the horizontal axis and oxygen tension as percent atmosphere on the vertical axis. One minute before mark 9 seven drops of TRUSOPT® (dorzolamide 2%) is applied to the eye.

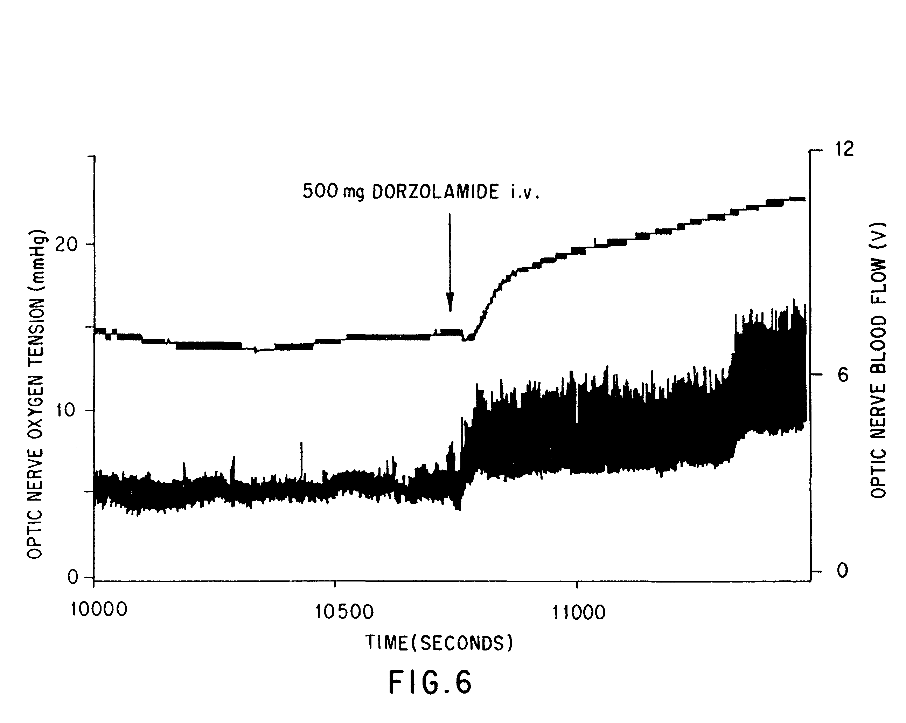

Figure 6. The effects of 500 mg intravenous dorzolamide on the optic nerve oxygen tension (expressed as mmHg on the left vertical axis) and the optic nerve blood flow (expressed as volts on the right vertical axis), recorded simultaneously from the same optic nerve. Time from the beginning of recording from this optic nerve is shown on the horizontal axis (in seconds).

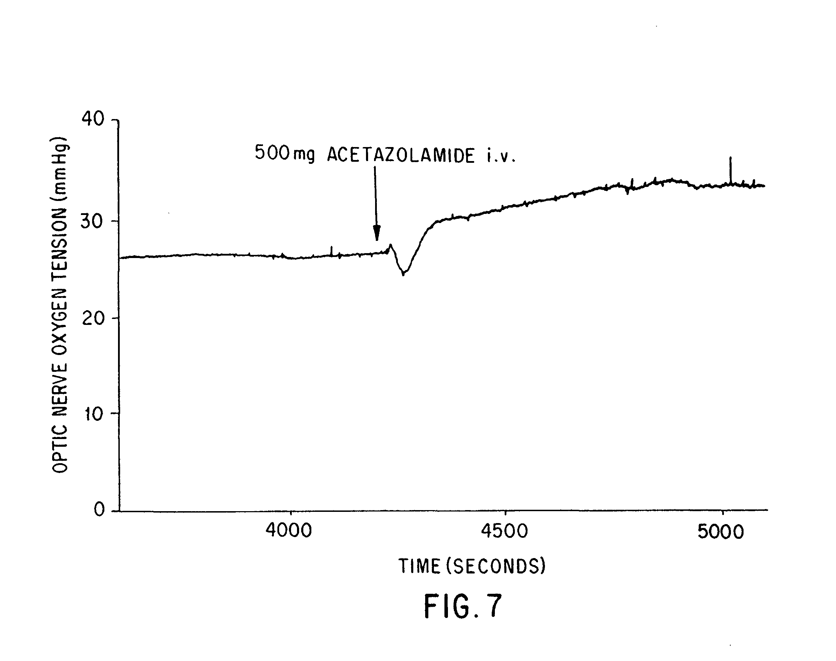

Figure 7. The effect of intravenous acetazolamide on optic nerve oxygen tension. Time from beginning of record is shown (in seconds) on the horizontal axis and oxygen tension (as mmHg) on the vertical axis. At the point indicated by the arrow an intravenous injection of 500 mg acetazolamide was given.

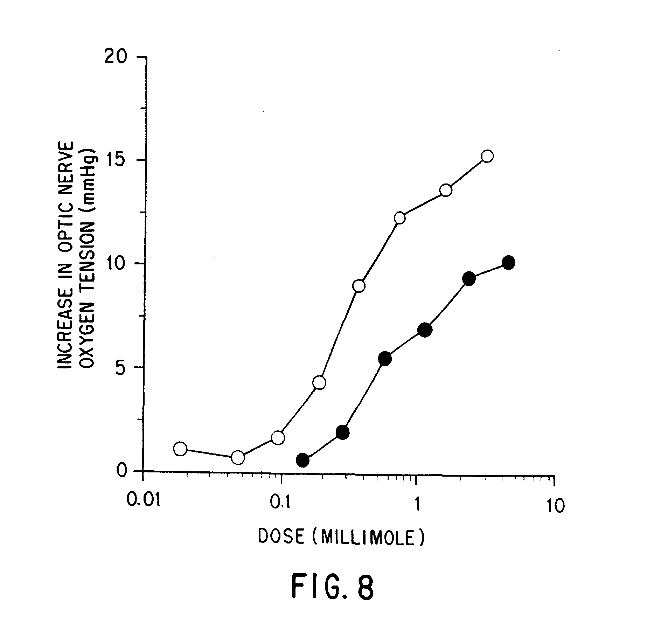

Figure 8. Dose-response relationship between intravenous acetazolamide (closed circles) and dorzolamide (open circles), expressed in log millimole, and increase in optic nerve oxygen tension (expressed as change in mmHg).

### SUMMARY OF THE INVENTION

The present invention is directed to a medicament for increasing retinal and optic nerve oxygen tension in a patient in need thereof comprising a carbonic anhydrase inhibitor (CAI) either orally, intraveneously or topically. A preferred medicament is a topical carbonic anhydrase inhibitor. The invention is also directed to a medicament for increasing retinal and optic nerve oxygen tension in a patient in need thereof comprising a hypotonic solution of xanthan gum and a carbonic anhydrase inhibitor, preferably a hypotonic solution of xanthan gum and a topical carbonic anhydrase inhibitor.

Particularly, the invention relates to a medicament for increasing retinal and optic nerve oxygen tension comprising a carbonic anhydrase inhibitor(CAI) such as those which are described in U.S. Patent Nos. 4,797,413, 4,386,098, 4,416,890, 4,426,388, 5,378,703, 5,240,923 and 5,153,192; Particularly preferred carbonic anhydrase inhibitors are topical carbonic anhydrase inhibitors belonging to the class having structural formula (I): 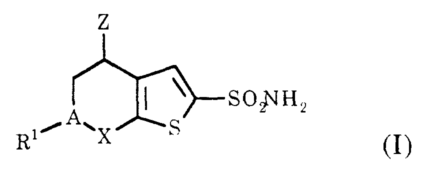 an individual diastereomer, an individual enantiomer or mixture thereof, or an ophthalmologically acceptable salt thereof, wherein:
A is carbon or nitrogen, preferably carbon;
Z is -NHR or -OR, preferably -NHR;
R is C₁₋₆alkyl, either straight or branched chain, preferably C₂₋₄alkyl such as ethyl, propyl or isobutyl;
R¹ is
   (a) hydrogen,
   (b) C₁₋₃alkyl, preferably methyl, ethyl or n-propyl, or
   (c) C₁₋₄alkoxy-C₁₋₄alkyl, preferably 3-methoxypropyl or ethoxymethyl; and
X is -S(O)₂- or -C(O)-, preferably -S(O)₂-.

The carbon atoms to which Z and R¹ are bonded may be chiral. When named according to absolute configuration, e.g., (R,S) or *(S,S),* the first letter represents the chirality the carbon atom to which Z is bonded and the second letter represents the chirality of A when A is carbon. The carbonic anhydrase inhibitors of this invention accordingly may be used as diastereomeric mixtures or single enantiomers or as racemic mixtures. More preferred carbonic anhydrase inhibitors are dorzolamide, brinzolamide, acetazolamide, metazolamide, sezolamide described in U.S. Patent Nos., 4,797,413, 4,386,098, 4,416,890, 4,426,388, 5,378,703, 5,240,923 and 5, 153; 192.

Dorzolamide, which has recently been approved under the trademark, TRUSOPT®, is the first topically effective CAI for clinical use in treating glaucoma and ocular hypertension. (S,S)-(-)-5,6-Dihydro-4-ethylamino-6-methyl-4*H*-thieno[2,3-b]thiopyran-2-sulfonamide-7,7-dioxide hydrochloride (dorzolamide HCl; MK507) is the active ingredient in TRUSOPT® which is prescribed for the treatment of elevated intraocular pressure in ocular hypertension, open-angle glaucoma and pseudo-exfoliative glaucoma. TRUSOPT® Ophthalmic Solution is applied as an isotonic, buffered, slightly viscous, aqueous solution of dorzolamide HCl. Each ml of TRUSOPT® 2% contains 20 mg dorzolamide (22.3mg dorzolamide HCl). When used as monotherapy, the dose is one drop of TRUSOPT® Ophthalmic Solution in the conjunctival sac of each affected eye three times daily.

More particularly, the invention relates to a médicament for increasing retinal and optic nerve oxygen tension by topical application of a composition wherein the topical carbonic anhydrase inhibitor is (*S,S*)-(-)-5,6-dihydro-4-ethylamino-6-methyl-4*H*-thieno[2,3-b]thiopyran-2-sulphonamide-7,7-dioxide, or an ophthalmologically acceptable salt thereof or 2H-thieno[3,2-e]-1,2-thiazine-6-sulfonamide-4-(ethylamino)-3,4-dihydro-2-(3-methoxypropyl)-1,1-dioxide or an ophthalmologically acceptable salt thereof. Other aspects of the invention will become apparent upon review of the complete application.

The present invention is directed to a medicament for increasing retinal and optic nerve oxygen tension by application of a carbonic anhydrase inhibitor and preferably by application of a topical carbonic anhydrase inhibitor. A more preferred medicament is a composition comprising a hypotonic solution of xanthan gum and a topical carbonic anhydrase inhibitor.

The present invention is based upon the finding that CAIs can preserve or benefit vision by increasing oxygen tension in the retina and optic nerve which when coupled with the attending increase in ocular blood flow benefits optic nerve health.

Research was done using dorzolamide and acetazolamide as the carbonic anhydrase inhibitors. It is a known compound useful as a carbonic anhydrase inhibitor and for the reduction of intraocular pressure.

In the compositions of the present invention the concentration of xanthan gum comprises about 0.1 to 2% (w/w), preferably 0.4 to 0.7% (w/w). Particularly preferred is KELTROL™T xanthan gum from Monsanto Performance Materials.

Xanthan gum is a high molecular weight polysaccharide gum obtainable from the aerobic fermentation of a carbohydrate with bacteria of the genus *Xanthomonas,* especially *Xanthomonas campestris.* Each xanthan gum repeat unit contains five sugar residues: two glucose, two mannose and one glucuronic acid. The polymer backbone consists of four β-D-glucose units linked at the 1 and 4 positions. Trisaccharide side chains on alternating anhydroglucose units distinguish xanthan gum from cellulose. Each side chain comprises a glucuronic acid residue between two mannose units. At most of the terminal mannose units is a pyruvate moiety.

Xanthan gum solutions are pseudoplastic. In other words, when shear stress is increased, the viscosity is progressively reduced. Upon reduction of the shear, total viscosity is recovered almost instantaneously. This behavior results from the high-molecular-weight molecule which forms complex molecular aggregates through hydrogen bonds and polymer entanglement. Also, this highly ordered network of entangled, stiff molecules accounts for the high viscosity observed at low shear rates. Shear thinning results from disaggregation of this network and alignment of individual polymer molecules in the direction of shear force. However, when the shearing ceases, aggregates re-form rapidly. As a result of its helical conformation, xanthan gum viscosity is relatively insensitive to temperature changes below the transition temperatures and to differences in ionic strength and pH. Xanthan gum solutions do not therefore have any liquid-gel phase transition properties, hence xanthan gum is not suitable for use in the formulation of *in situ* gelling solutions.

The results of the present invention suggest that the high degree of pseudoplasticity, which is independent of concentration, appears to be important in contributing to the unusual ocular penetration properties of the hypotonic formulation of xanthan gum.

Xanthan gum is commercially available, for example, under the tradename KELTROL^{TM} from Monsanto Performance Materials, a unit of Monsanto Company, St. Louis, MO 63167, USA.

Following administration of the composition to the conjunctival sac of a patient's eye, there is no liquid-gel phase transition. The advantageous enhancement in ocular bioavailability of the topical carbonic anhydrase inhibitor is achieved through the unique combination of the properties of the hypotonic solution of xanthan gum.

The topical carbonic anhydrase inhibitors of use in the present invention include those compounds described in U.S. Patent Nos. 4,797,413, 4,386,098, 4,416,890. 4,426,388, 5,378,703, 5,240,923 and 5,153,192. Preferred topical carbonic anhydrase inhibitors are the class of compounds of the structural formula (I): 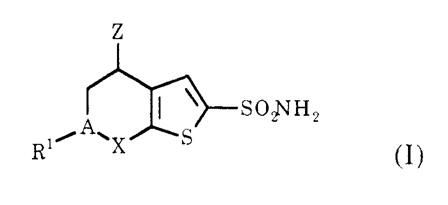 an individual diastereomer, an individual enantiomer or mixture thereof, or an ophthalmologically acceptable salt thereof. wherein:
A is carbon or nitrogen, preferably carbon;
Z is -NHR or -OR, preferably -NHR;
R is C₁₋₆alkyl, either straight or branched chain, preferably C₂₋₄alkyl such as ethyl, propyl or isobutyl;
R¹ is
   (a) hydrogen,
   (b) C₁₋₃alkyl, preferably methyl, ethyl or n-propyl, or
   (c) C₁₋₄alkoxy-C₁₋₄alkyl, preferably 3-methoxypropyl or ethoxymethyl; and
X is -S(O)₂- or -C(O)-, preferably -S(O)₂-.

The carbon atoms to which Z and R¹ are bonded may be chiral. When named according to absolute configuration, e.g., (R,S) or (*S*,*S*), the first letter represents the chirality the carbon atom to which Z is bonded and the second letter represents the chirality of A when A is carbon. The carbonic anhydrase inhibitors of this invention accordingly may be used as diastereomeric mixtures or single enantiomers or as racemic mixtures.

Preferred topical carbonic anhydrase inhibitors for use in the present invention are compounds of formula (1), above, wherein A is carbon; and wherein R is -CH₂CH₃ and R¹ is -CH₃; or R is -CH₂CH₂CH₃ and R¹ is -CH₂CH₂CH₂OCH₃; or R is -CH₂CH₃ and R¹ is -CH₂CH₂CH₃; or R is -CH₂CH₂(CH₃)₂ and R¹ is hydrogen; or R is -CH₂CH₃ and R¹ is -CH₂OCH₂CH₃; and carbons 4 and 6 of the topical carbonic anhydrase inhibitor both have S absolute stereochemical configuration.

More preferred carbonic anhydrase inhibitors are dorzolamide, brinzolamide, acetazolamide, metazolamide described in U.S. Patent Nos., 4,797,413, 4,386,098, 4,416,890, 4,426,388, 5,378,703, 5,240,923 and 5,153,192. Dorzolamide, which has recently been approved under the trademark, TRUSOPT®, is the first topically effective CAI for clinical use.

Most preferred topical carbonic anhydrase inhibitors are (S,S)-(-)-5,6-dihydro-4-ethylamino-6-methyl-4H-thieno[2,3-b]thiopyran-2-sulphonamide-7,7-dioxide, or an ophthalmologically acceptable salt thereof and 2H-thieno[3,2-e]-1,2-thiazine-6-sulfonamide-4-(ethylamino)-3,4-dihydro-2-(3-methoxypropyl)-1,1-dioxide or an ophthalmologically acceptable salt thereof.

A particularly preferred topical carbonic anhydrase inhibitor is dorzolamide, especially as its hydrochloride salt.

The concentration of TCAI in the present invention is 0.05 to 5% (w/w), usually 0.5 to 3% (w/w), and is administered one to three times daily, preferably one to two times daily, to each affected eye.

The novel medicament can be administered by the topical ocular administration of 0.025 to 5 mg per day, preferably 0.25 to 3 mg per day, of the topical carbonic anhydrase inhibitor to each eye. Alternatively, it can be administered by intravenous administration of 5 mg to 600 mg of the carbonic anhydrase inhibitor to each affected eye.

As a unit dosage, between 0.025 and 2.5 mg, preferably between 0.25 and 1.5 mg, of the topical carbonic anhydrase inhibitor is applied to each eye. Alternatively, as a unit dosage, between 5 mg to 250 mg, of the carbonic anhydrase inhibitor is applied intravenously to each eye.

Conventional ophthalmic solutions are usually prepared as isotonic solutions using tonicity adjusting agents such as potassium chloride, sodium chloride, mannitol, dextrose and glycerin. An isotonic solution will have a freezing point depression of approximately -0.54°C. Tonicity may also be measured by the osmolality of the solution, an isotonic solution having an osmolality of about 290 milliosmoles per kilogram (mOs/kg).

It is a characteristic of the ophthalmic compositions of the present invention that they are hypotonic solutions, with a freezing point depression between -0.28°C and -0.4°C, and preferably between -0.31 °C and -0.37°C.

Alternatively, the hypotonicity of the ophthalmic solutions of the present invention is between 150 and 215 mOs/kg, and preferably between 170 and 200 mOs/kg.

According to a further aspect of the present invention, there is provided a novel medicament for increasing retinal and optic nerve oxygen tension by application of a carbonic anhydrase inhibitor, preferably by topical application of a composition comprising a hypotonic solution of xanthan gum, a therapeutically effective amount of a topical carbonic anhydrase inhibitor and a therapeutically effective amount of a β-adrenergic receptor blocking agent. Suitable β-adrenergic receptor blocking agents include betaxolol, bufetolol, carteolol, levobunolol, metipranolol, and timolol, or an ophthalmologically acceptable salt thereof. A particularly preferred β-adrenergic receptor blocking agent is timolol maleate.

Such compositions preferably comprise 0.05 to 5% (w/w) of the topical carbonic anhydrase inhibitor, usually 0.5 to 3% (w/w), preferably 0.7% to 2.0% (w/w) and 0.01 to 1% (w/w) of the β-adrenergic receptor blocking agent, preferably 0.1 to 0.5% (w/w) to be administered once or twice a day to each affected eye.

Thus, the medicament can further be administered by the topical ocular administration of 0.025 to 5 mg per day, preferably 0.25 to 3 mg per day, of the topical carbonic anhydrase inhibitor and 0.005 to 1 mg per day, preferably 0.05 to 0.5 mg per day, of the β-adrenergic receptor blocking agent to each eye.

As a unit dosage, between 0.005 and 0.5 mg of the ß-adrenergic receptor blocking agent, and preferably between 0.05 and 0.25 mg of the β-adrenergic receptor blocking agent, is applied to each eye.

The pH of the composition ranges from 5.0 to 7.5, preferably 5.5 to 7.0.

According to a further aspect of the present invention, there is provided a novel medicament for increasing retinal and optic nerve oxygen tension by application of a carbonic anhydrase inhibitor, preferably by application of a composition comprising a hypotonic solution of xanthan gum, a therapeutically effective amount of a topical carbonic anhydrase inhibitor and a therapeutically effective amount of a prostaglandin, prostaglandin derivative or pharmaceutically acceptable salt thereof, such as those described in U.S. Patent 4,883,819, 4,824,857, 5.001,153 and 4,599,353. Suitable prostaglandins include 13, 14-dihydro-15(R)-17-phenyl-18, 19, 20-trinor-PGF2α esters, or 13, 14-dihydro-15-keto-20-ethyl-PGF2α isopropryl esters, or an ophthalmologically acceptable salt thereof. Preferred prostaglandins are:
11-pivaloyl prostaglandin F2α hydroxyethyl ester,
(+)-(Z)-sodium-7-[1R, 2R, 3R, 5S)-3,5-dihydroxy-2-[(E)-1-octenyl]cyclopentyl]-5-heptenoate sesquihydrate,
[1α,2β,3α,5α]methyl-5-cis-2-(phenylethylsulfonamidomethyl)-3,5-dihydroxycyclopentyl heptenoate,
(+-)-5-[6-(1-hydroxy)hexyl)-1,3-benzodioxol-5-yl]-pentanol, 15-pivaloyl PGFα,
7-[3α,5α dihydroxy-2-(3a-hydroxy-5--1E-pentenyl)cyclopentyl]-5Z-heptenoic acid,
isopropyl (Z)-7-[(1R,2R,3R,5S)-3,5-dihydroxy-2-[(3R)-3-hydroxy-5-phenylpentyl]cyclopentyl]-5-heptenoate or
13,14-dihydro-15-keto-20-ethyl-PGF2α isopropyl ester trimethylphenol-1-acetate.

Most preferred prostaglandins are isopropyl (Z)-7-[(1R,2R,3R,5S)-3,5-dihydroxy-2-[(3R)-3-hydroxy-5-phenylpentyl]cyclopentyl]-5-heptenoate, (+)-(Z)-sodium-7-[1R, 2R, 3R, 5S)-3,5-dihydroxy-2-[(E)-1-octenyl]cyclopentyl]-5-heptenoate sesquihydrate, or 13,14-dihydro-15-keto-20-ethyl-PGF2α isopropyl ester trimethylphenol-1-acetate

The ophthalmic formulations of this invention comprise 0.025 to 5% (w/w) of the carbonic anhydrase inhibitors discussed herein, preferably 5,6-dihydro-4-ethylamino-6-methyl-4H-thieno-[2,3-b]thiopyran-2-sulfonamide-7,7 dioxide hydrochloride or 2H-thieno[3,2-e]-1,2-thiazine-6-sulfonamide-4-(ethylamino)-3,4-dihydro-2-(3-methoxypropyl)-1,1-dioxide and their trans and cis enantiomers, or an ophthalmologically acceptable salt thereof, including racemic material, usually 0.5 to 3% (w/w) and more preferably 0.7 to 2% (w/w) and 0.005 to 2.0% (w/w), preferably 0.1 to 1% (w/w) of the prostaglandin or prostaglandin derivatives discussed herein, preferably 13,14-dihydro-15(R)-17-phenyl- 18, 19, 20-trinor-PGF2a esters or 13, 14-dihydro-15-keto-20-ethyl-PGF2α isopropryl esters, and more preferably isopropyl (Z)-7-[(1R,2R,3R,5S)-35-dihydroxy-2-[(3R)-3-hydroxy-5-phenylpentyl]cyclopentyl]-5-heptenoate, or 13,14-dihydro-15-keto-20-ethyl-PGF2α isopropyl ester trimethylphenol-1-acetate, to be administered on a 1 to 2 times a day schedule.

The medicament of this invention can be administered by the topical ocular administration of 0.025 to 5 mg per day, preferably 0.25 to 3 mg per day of a carbonic anhydrase inhibitor and concomitant, prior, or previous administration of 0.005 to 2 mg per day, preferably 0.1 to 1.0 mg per day, of prostaglandin or prostaglandin derivative to each eye, 1 to 2 times a day.

The medicament is suitable for primates, man and other animals, particularly man and domesticated animals such as cats and dogs.

The pharmaceutical preparation may contain non-toxic auxiliary substances such as antibacterial components which are non-injurious in use, for example, thimerosal, benzalkonium chloride, methyl and propyl paraben, benzyldodecinium bromide, benzyl alcohol, or phenylethanol; buffering ingredients such as sodium chloride, sodium borate, sodium acetate, sodium citrate, or gluconate buffers; and other conventional ingredients such as sorbitan monolaurate, triethanolamine, polyoxyethylene sorbitan monopalmitylate, ethylenediamine tetraacetic acid.

The following examples of ophthalmic compositions are given by way of illustration.

### EXAMPLE 1

| SOLUTION COMPOSITION | | A | B |
|---|---|---|---|
| *(S,S)-(-)-5,6-Dihydro-4-ethylamino-6- methyl-4H-thieno[2,3-b]thiopyran-2-* sulfonamide-7,7-dioxide monohydrochloride | | 2.226% | 2.226% |
| Sodium Chloride | | 0.2% | 0.2% |
| Benzalkonium Chloride | | 0.0075% | 0.0075% |
| Sodium hydroxide | QS | pH 5.6 | pH 5.6 |
| Purified Water | QS | 100% | 100% |

The active compound, sodium chloride and benzalkonium chloride were dissolved in water for injection. The pH of the composition was adjusted to 5.6 by addition of 0.2 N sodium hydroxide solution, and water for injection was added until the weight of composition was equal to 75 parts of the final weight (Example 1A) or 65 parts of the final weight (Example 1B). The composition was sterilised by filtration, and the solution flushed with sterile nitrogen. The solution was aseptically subdivided into sterile vials and sealed.

### Example 2

| SOLUTION COMPOSITION | | A | B |
|---|---|---|---|
| (S,S)-(-)-5,6-Dihydro-4-ethylamino-6-methyl-4H-thieno[2,3-b]thiopyran-2-sulfonamide-7,7-dioxide monohydrochloride | | 2.226% | 2.226% |
| Xanthan Gum | | 0.5% | 0.7% |
| Sodium Chloride | | 0.2% | 0.2% |
| Benzalkonium Chloride | | 0.0075% | 0.0075% |
| Sodium hydroxide | QS | pH 5.6 | pH 5.6 |
| Purified Water | QS | 100% | 100% |

The active compound, sodium chloride and benzalkonium chloride were dissolved in water for injection. The pH of the composition was adjusted to 5.6 by addition of 0.2 N sodium hydroxide solution, and water for injection was added until the weight of composition was equal to 75 parts of the final weight (Example 2A) or 65 parts of the final weight (Example 2B). The composition was sterilised by filtration, and the solution flushed with sterile nitrogen. Then a clarified, steam sterilised concentrate of 2% xanthan gum was added to the solution of drug and the obtained solution was homogenised by stirring. The solution was aseptically subdivided into sterile vials and sealed.

### EXAMPLE 3

| SOLUTION COMPOSITION | | |
|---|---|---|
| (S, S)-(-)-5,6-dihydro-4-ethylamino-6-methyl-4H-thieno[2,3-b]thiopyran-2-sulfonamide-7,7-dioxide monohydrochloride | | 2.226% |
| (*S*)-(-)-1-(*tert*-butylamino)-3-[(4-morpholino-1,2,5-thiadiazol-3-yl)oxy]-2-propanol maleate | | 0,684% |
| Xanthan Gum | | 0.5% |
| Sodium Chloride | | 0.15% |
| Sodium Hydroxide | QS | pH 5.6 |
| Benzalkonium Chloride | | 0.0075% |
| Purified Water | QS | 100% |

The active compounds, sodium chloride and benzalkonium chloride are dissolved in water for injection. The pH of the composition is adjusted to pH 5.6 by addition of sodium hydroxide, and water for injection is added until the weight of the composition was equal to 75 parts of the final weight. The composition is sterilised by filtration, flushing with sterile nitrogen. Then a clarified, steam sterilised concentrate of 2% xanthan gum is added to the solution of the drug and the obtained solution is homogenised by stirring. The solution is aseptically subdivided into sterile vials and sealed.

### Example 4

### Examining Oxygen Tension of Optic Nerve(ONPO₂)

Danish agricultural pigs (landrace), 25-33 kg in weight and brought up in specific pathogen free environment, were used. Their treatment was supervised by a veterinarian and followed the decrees of the ARVO Statement for the use of animals in ophthalmic and vision research. All experiments were conducted during daytime. The pigs were anaesthetised with midazolam (dormicum®) and ketamine followed by intravenous application of 250 mg mebumal, fentanylum (Haldid®, Jansen Cilag), 0.5 mg an hour, pancuron bromide (Pavulon®, Organon Technica, Sweden) 16 mg an hour, mebumal (Hospital Pharmacies, Denmark) 50 mg per ml as needed and atropine as needed. The right pupil was dilated with 1% tropicamide eye drops (Mydriacyl® and 2.5% phenylephrine eye drops. A arterial line was placed in a femoral artery for continuous measurement of blood pressure and intermittent blood gas analysis. A venous line was placed in a femoral vein for continuous infusion of the anaesthetics and saline as needed. A rectal thermoprobe provided continuous reading of body temperature.

The pig was secured laterally on an operating table and a speculum was placed between the right eyelids. A conjunctival peritomy was performed and 3-0 silk sutures placed under the rectus muscle tendons to immobilise the eye. A sclerotomy was made 2.5 mm behind the limbus in the superonasal quadrant and a cannula placed, through which the polarographic electrode was advanced into the vitreous cavity. The electrode was held in a micromanipulator (Stoelting Inc. USA) and placed in the vitreous humour approximately 0.5 mm over the optic disc in an area not covered by the large vessels. The entire operation was performed using a Zeiss operating microscope and the electrode was placed using an indirect ophthalmoscope or the microscope and a flat corneal contact lens.

A silver-silver chloride reference electrode was placed in the conjunctival sac of the same (right) eye and kept moist with saline. The polarographic oxygen meter consists of a 100 micrometer platinum-iridium electrode inside a 20 gauge needle (Model 761), a silver-silver chloride reference electrode and a chemical microsensor (Model 1251, Diamond Electrotech Inc., Ann Arbor Mich., USA). The recording system was calibrated before and after each experiment in a calibration cell (Diamond Electrotech. Inc.) at 37 degrees Celsius in 0.9% saline using 100% N₂, 5.0%0₂-95%N₂ and 20.000%0₂-80%N₂ (provided by AGA Inc. Denmark). The barometric pressure was monitored, and the oxygen tension recordings were adjusted for barometric pressure, and expressed as mmHg.

The experiments were all run with typical indoor illumination (white fluorescent light tubes (Philips TLM 4OW/29RS)). The ambient light intensity at the level of the pig's eye was measured with a Mavolux digital light meter (Gossen GmbH, Germany) and found to be 497 lux or 6 watt per square meter.

Arterial blood samples were obtained at regular intervals and arterial PO₂, pH and PCO₂ were measured with a ABL 605 blood gas analyser (Radiometer, Denmark) (table 1). Arterial blood pressure, electrocardiogram and rectal temperature were monitored continuously and it was verified that these values were within normal limits. The output of the PO₂ electrometer, together with continuous recordings of the arterial blood pressure and the electrocardiogram, was digitised on line with a MacLab 4/e analog-digital converter (AD Instruments Pty., Australia). The digitised recordings were fed into a power Macintosh computer. Data were displayed and analysed with the Chart v3.5.4/s software (AD Instruments Pty, Australia) and printed on an Apple LaserWriterII printer.

Each experiment was begun with the animal breathing 100% oxygen. Once a stable ONPO₂ level was obtained the breathing mixture was changed to air, containing 21% oxygen, and a stable level obtained again. In some experiments the breathing mixture was changed to 20.000% O₂ followed by 5.19% CO₂, 19.9% O₂ (N₂ to balance), followed by 20.000% O₂. The test drug was then administered by intravenous injection into the femoral line. Saline injections (20 ml intravenous) were done as controls. Dorzolamide (4%, MSD Inc., 6-500 mg doses) or acetazolamide (Diamox, Lederle Inc.) 15-500 mg were injected intravenously.

A laser doppler probe (Periflux 4001, Master, Perimed AB, Sweden) for measurement of blood flow was placed through the pars plana transvitreally onto the optic disc near the oxygen electrode, to obtain simultaneous recordings of optic nerve oxygen tension and blood flow. In three experiments the intraocular pressure was fixed at 20 cm of saline (15 mmHg) with a needle in the anterior chamber connected to a saline reservoir, and recorded with a pressure transducer, while dorzolamide was injected intravenously and ONPO₂ recorded. Means and standard deviations were calculated and differences evaluated with a paired Student's t-test.

### Results

The effects of inhaled O₂ and CO₂.

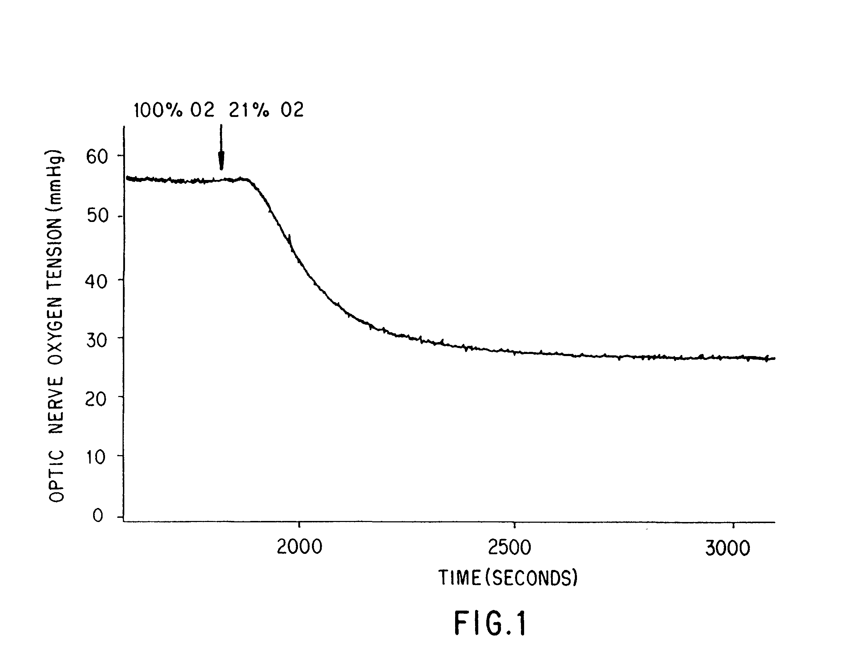

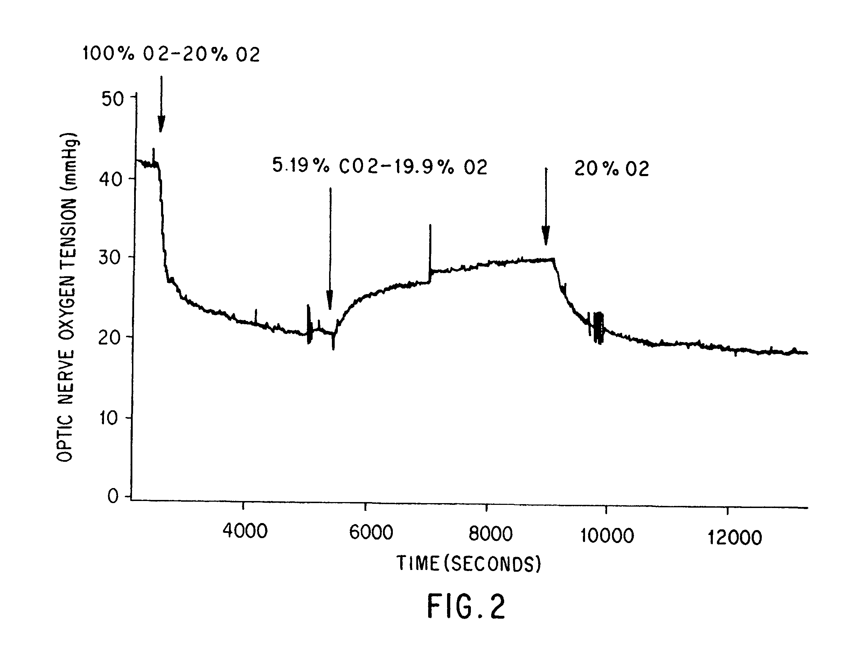

In the initial set of experiments the oxygen tension at the optic nerve head while the animals inhaled various concentrations of oxygen ,and CO₂, was examined. Stable, continuous and reproducible recordings of ONPO₂ were obtained for up to 8 hours in pigs. The mean ONPO₂ was 24.1 ± 11.6 mmHg when breathing room air, and 50.7 ± 29.3 mmHg while the animals were breathing 100% 0₂, (n=15; p<0.001). Figure 1 shows an experinient were the animal first inhaled 100% 0₂. When the oxygen content was reduced to 21 % 0₂, there was an immediate reduction in the ONPO₂, In four experiments the breathing mixture was switched from 20% O₂ to a mixture of 5.19% CO₂ and 19.9% O₂ (N₂ to balance). In response to breathing 5.19% CO₂ the ONPO₂ changed from 20.8 ± 5.6 mmHg to 28.9 ± 3.6 mmHg (n= 4, p<0.001). An example of this increase in ONPO₂ is illustrated in Figure 2, which also shows that almost immediately upon returning to 20% O₂-80% N₂, the ONPO₂ returned to its value before breathing CO₂. As shown in Table 2, CO₂ breathing significantly lowered the arterial pH, and also raised the arterial PCO₂, but had no significant effect on the arterial PO₂.

### The effects of carbonic anhydrase inhibition.

In another set of experiments the effects of inhibiting carbonic anhydrase activity by intravenous injection of two carbonic anhydrase inhibitors, commonly used as treatment for glaucoma were examined. Polarographic oxygen electrodes were placed in the vitreous humour 0.5 mm above the optic disc in anaesthetized pigs. Arterial blood pressure and gasses were monitored and ONPO was recorded continously with various breathing gasses. The test drug was than administered by intravenous injection in the femoral line or by topical application on the eye. Saline injections (20 ml intravenous) or topical application on the eye were done as controls. Dorzolamide (4% MSD Inc., 500, 250, or 125 mg) was injected intravaneously or topical on the eye was 4% solution or 2% (TRUSOPT®, MSD, Inc.) solution. Acetazolamide (Diamox Lederle Ind.) 500 mg, 250 mg, 125 mg or 31 mg were injected intravenously. The carbonic anhydrase inhibitors, dorzolamide and acetazolamide raised the optic nerve oxygen tension by about 25-60% (n=4, p=0.046) and 15-35% (n=4, p=0.005), respectively.

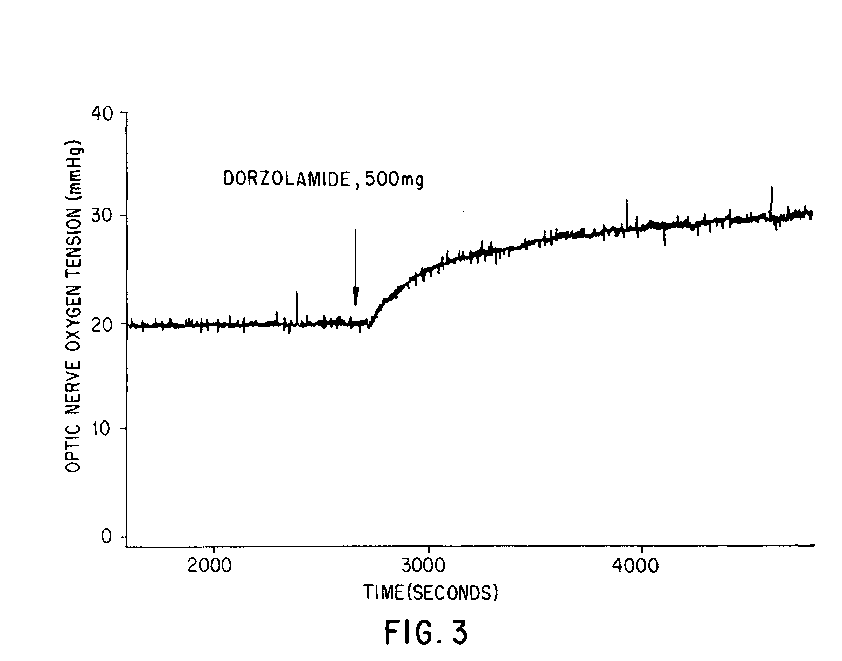

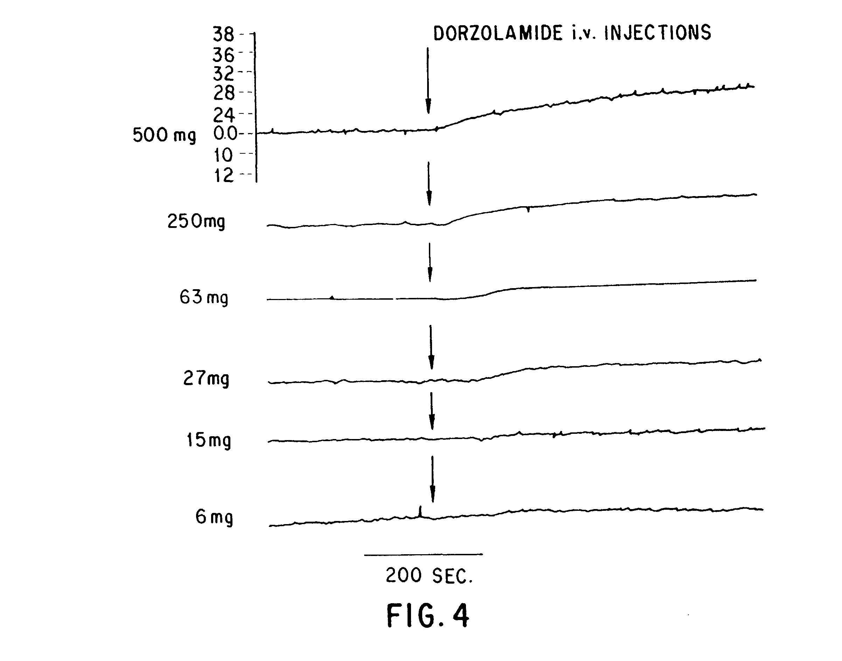

Specifically, intravenous injections of 500 mg dorzolamide raised the ONPO₂ from 16.4 ± 6.1 mmHg to 26.9 ± 12.2 mmHg, or 52.5% ± 21.2% (n=5; p= 0.017), as illustrated by a typical expetiment (Figure 3). As shown in Table 2, dorzolamide significantly lowered the arterial pH, and also raised the arterial PCO₂, but had no significant effect on the arterial PO₂. The effect of dorzolamide on ONP0₂ is dependent on the dose injected, and an effect is seen with injections of 6 to 500 mg (Figure 4). The magnitude and time course of the response is related to dosage. Figure 5 shows the results of topical administration. One minute before mark 9 seven drops of TRUSOPT® (dorzolamide 2%) is applied to the eye.

In some experiments, optic nerve blood flow was recorded concurrently with ONPO2, with a laser Doppler probe (Figure 6). In this experiment 500 mg dorzolamide was injected intravenously. Both ONPO2 and optic nerve blood flow rose simultaneously shortly after infusion of the drug.

Intravenous injections of 500 mg acetazolamide raised the ONPO2 from 23.6± 9.5 mmHg to 30.9± 10.0 mmHg(n=6, p=0.0008). Figure 7 shows a typical response to intravenous injection of acetazolaimde. A transient lowering of arterial blood pressure follows the acetazolamide injection and corresponds in time to the transient dip in the ONPO2 recording. Control injections and topical application of saline had no effect on ONPO2.

The effects of acetazolamide and dorzolamide on ONPO2 are dose dependent, and dorzolamide is more potent as demonstrated by Figure 8.

**Table 1.**

| physiological parameters in experimental pigs | | | | |
|---|---|---|---|---|
| Parameter | Mean | st. dev. | max, | min. |
| Heart rate (per minute) | 113.6 | 33.2 | 187 | 65 |
| Blood pressure (mmHg) | 100.9 | 16.2 | 127 | 75 |
| pH | 7.61 | 0.042 | 7.66 | 7.49 |
| pCO₂ (kPa): | 4.04 | 0.471 | 5.55 | 3.3 |
| pO₂ in air (kPa) | 13.67 | 1.46 | 17.43 | 10.57 |
| pO₂ in 100%0₂ (kP&) | 70.13 | 4.85 | 76.45 | 61.54 |
| Rectal Temp (C) | 38.28 | 1.09 | 40.7 | 36.7 |
| Electrode drift (%O₂ per hour) | 0.033 | 0.061 | 0.214 | 0.006 |

**Table 2.**

| Alterations in arterial blood gases | | | | | | | | | |
|---|---|---|---|---|---|---|---|---|---|
| | Before Mean | SD | After Mean | SD | Before Mean | SD | After Mean | SD | Before Mean |
| CO₂ breathing acetazolamide | 7,62 | 0,05 | 7,42 | 0,03* | 13,84 | 1,1 | 14,13 | 0,93 | 3,96 |
| 500 mg dorzolamide | 7,62 | 0,01 | 7,49 | 0,03* | 14,79 | 1,67 | 13,62 | 1,5 | 3,84 |
| 500 mg | 7,57 | 0,05 | 7,43 | 0,05* | 12,96 | 1,58 | 12,36 | 0,7 | 4,42 |

| | | | | | | | | | |
|---|---|---|---|---|---|---|---|---|---|
| *p<0.001 | | | | | | | | | |

## Claims

1. Use of a carbonic anhydrase inhibitor for the manufacture of a medicament for applying to the eye for increasing the optic nerve and retinal oxygen tension, excepting the treatement of glaucoma.

2. The Use according to claim 1 wherein the medicament comprises a hypotonic solution of 0.1 to 2% (w/w) of xanthan gum and 0.5 to 5% (w/w) of a carbonic anhydrase inhibitor.

3. The Use according to Claim 2 wherein the carbonic anhydrase inhibitor is a compound of the structural formula (I): 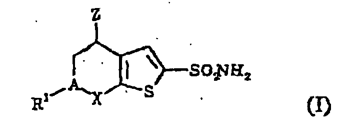 an individual diastercomer, an individual enantiomer or mixture thereof, or an ophthalmologically acceptable salt thereof, wherein:
A is carbon or nitrogen, preferably carbon;
Z is -NHR or -OR, preferably -NHR;
R is C₁₋₆alkyl, either straight or branched chain, preferably C₂₋₄alkyl such as ethyl, propyl or isobutyl;
R¹ is
(a) hydrogen,
(b) C₁₋₃alkyl, preferably methyl, ethyl or n-propyl, or
(c) C₁₋₄alkoxy-C₁₋₄alkyl, preferably 3-methoxypropyl or ethoxymethyl; and
X is -S(O)₂- or -C(O)-, preferably -S(O)₂-.

4. The use according to Claim 3 wherein A is carbon; and wherein R is -CH₂CH₃ and R¹ is -CH₃; or R is -CH₂CH₂CH₃ and R¹ is -CH₂CH₂CH₂OCH₃; or R is -CH₂CH₃ and R¹ is -CH₂CH₂CH₃; or R is -CH₂CH₂(CH₃)₂ and R¹ is hydrogen; or R is -CH₂CH₃ and R¹ is -CH₂OCH₂CH₃; and carbons 4 and 6 of the topical carbonic anhydrase inhibitor both have S absolute stereochemical configuration.

5. The use according to Claim 2 or 4 wherein the carbonic anhydrase inhibitor is selected from the group consisting of dorzolamide, brinzolamide, acetazolamide, metazolamide and the like.

6. The use according to Claim 5 wherein the carbonic anhydrase inhibitor is dorzolamide.

7. The use method according to Claim6 wherein the dorzolamide is (*S,S'*)-(-)-5,6-dihydro-4-ethylamino-6-methyl-4*H* thieno(2,3-b]thiopyran-2-sulfonamide-7,7-dioxide hydrochloride.

8. The use anyone of claims 2 to 7 wherein the concentration of carbonic anhydrase inhibitor is 0.5 to 2.0% (w/w) and the concentration of xanthan gum is 0.4 to 0.7% (w/w).

9. The use of anyone of claims 2 to 8 wherein the composition is a hypotonic solution with a freezing point depression of between -0.28°C and -0:4°C and the hypotonicity of the solution is between 150 and 215 mOs/kg.

10. The use according to anyone of claims 1 to 9 wherein the medicament additionally comprises 0.01 to 1% (w/w) of a β-adrenergic receptor blocking agent, or an ophthalmologically acceptable salt thereof, for simultaneous, separate or sequential administration.

11. The use according to Claim to wherein the concentration of β-adrenergic receptor blocking agent is 0.1 to 0.5% (w/w).

12. The use according to Claim 11 wherein the β-adrenergic receptor blocking agent is timolol maleate.

13. The use according to claim 11 or 12 which additionally comprises 0.005 to 2.0% (w/w) of a prostaglandin, prostaglandin derivative, or an ophthalmologically acceptable salt thereof, for simultanous separate or sequestial administration.

14. The use method according to Claim 13 wherein the concentration of prostaglandin or prostaglandin derivative is 0.1 to 1% (w/w).

15. The use according to Claim 14 wherein the prostaglandin,)prostaglandin or derivative is:
11-pivaloyl prostaglandin F2α hydroxyethyl ester,
(+)-(Z)-sodium-7-[1R, 2R, 3R, 5S)-3,5-dihydroxy-2-[(E)-1-octenyl]cyclopentyl]-5-heptenoate sesquihydrate,
[1α,2β,3α,5α]methyl-5-cis-2-(phenylethylsulfonamidomethyl)-3,5-dihydroxycyclopentyl heptenoate,
(+-)-5-[6-(1-hydroxy)hexyl)-1,3-benzodioxol-5-yl]-pentanol, 15-pivaloyl PGFα,
7-[3α,5α dihydroxy-2-(3a-hydroxy-5--1E-penteny-1)cyclopentyl]-5Z-heptenoic acid,
isopropyl (Z)-7-[(1R,2R,3R,5S)-3,5-dihydroxy-2-((3R)-3-hydroxy-5-phenylpentyl)cyclopennyl]-5-hepteno ate or
13,14-dihydro-15-keto-20-ethyl-PGF2α isopropyl ester trimethylphenol-1-acetate.

16. The use according to Claim 15 wherein the prostaglandin or prostaglandin derivative is isopropyl (Z)-7-[(1R,2R,3R,5S)-3,5-dihydroxy-2-[(3R)-3-hydroxy-5-phenylpentyl]cyclopentyl]-5-heptenoate, (+)-(Z)-sodium-7-[1R, 2R, 3R, 5S)-3,5-dihydroxy-2-[(E)-1-octenyl]cyclopentyl]-5-heptenoate sesquihydrate, or 13,14-dihydro-15-keto-20-ethyl-PGF2α isopropyl ester trimethylphenol-1-acetate.

## Patentansprüche

1. Benutzung eines Carboanhydrasehemmers zur Herstellung eines Medikamentes zum Anwenden am Auge zum Erhöhen der Sauerstoffspannung im Sehnerven und in der Netzhaut, mit Ausnahme der Behandlung von Glaukom.

2. Benutzung nach Anspruch 1, wobei das Medikament eine hypotonische Lösung von 0,1 bis 2 Gew.% Xanthangummi und 0,5 bis 5 Gew.% eines Carboanhydrasehemmers umfaßt.

3. Benutzung nach Anspruch 2, wobei der Carboanhydrasehemmer eine Verbindung mit der Strukturformel (I) ist: 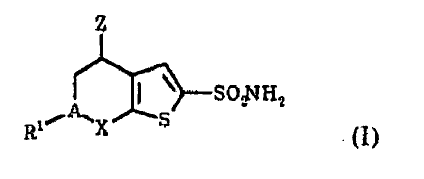 ein einzelnes Diastereomer, ein einzelnes Enantiomer oder ein Gemisch davon oder ein ophthalmologisch unbedenkliches Salz davon ist, wobei:
A Kohlenstoff oder Stickstoff, vorzugsweise Kohlenstoff, ist;
Z -NHR oder -OR, vorzugsweise -NHR, ist;
R C₁₋₆-Alkyl, entweder gerad- oder verzweigtkettig, vorzugsweise C₂₋₄-Alkyl, wie z.B. Ethyl, Propyl oder Isobutyl, ist;
R¹
(a) Wasserstoff,
(b) C₂₋₃-Alkyl, vorzugsweise Methyl, Ethyl oder n-Propyl, oder
(c) C₁₋₄-Alkoxy-C₁₋₄-Alkyl, vorzugsweise 3-Methoxypropyl oder Ethoxymethyl, ist; und
X -S(O)₂- oder -C(O)-, vorzugsweise -S(O)₂-, ist.

4. Benutzung nach Anspruch 3, wobei A Kohlenstoff ist; und wobei R -CH₂CH₃ ist und R¹ -CH₃ ist; oder R -CH₂CH₂CH₃ ist und R¹ -CH₂CH₂CH₂OCH₃ ist; oder R -CH₂CH₃ ist und R¹ -CH₂CH₂CH₃ ist; oder R -CH₂CH₂(CH₃)₂ ist und R¹ Wasserstoff ist; oder R -CH₂CH₃ ist und R¹ -CH₂OCH₂CH₃ ist; und die Kohlenstoffatome 4 und 6 des topischen Carboanhydrasehemmers beide eine absolute stereochemische S-Konfiguration aufweisen.

5. Benutzung nach Anspruch 2 oder 4, wobei der Carboanhydrasehemmer aus der Gruppe ausgewählt ist, die aus Dorzolamid, Brinzolamid, Acetazolamid, Metazolamid und dergleichen besteht.

6. Benutzung nach Anspruch 5, wobei der Carboanhydrasehemmer Dorzolamid ist.

7. Benutzung nach Anspruch 6, wobei das Dorzolamid *(S,S)-(-)-5,6-Dihydro-4-ethylamino-6-methyl-4H*-thieno[2,3-b]thiopyran-2-sulfonamid-7,7-dioxid-hydrochlorid ist.

8. Benutzung eines der Ansprüche 1 bis 7, wobei die Konzentration des Carboanhydrasehemmers 0,5 bis 2,0 Gew.% und die Konzentration von Xanthangummi 0,4 bis 0,7 Gew.% beträgt.

9. Benutzung eines der Ansprüche 2 bis 8, wobei die Zusammensetzung, wobei die hypotonische Lösung eine Gefrierpunktserniedrigung zwischen -0,28 °C und -0,4 °C aufweist und die Hypotonizität der Lösung zwischen 150 und 215 mOs/kg beträgt.

10. Benutzung nach einem der Ansprüche 1 bis 9, wobei das Medikament außerdem 0,01 bis 1 Gew.% eines β-Adrenorezeptorenblockers oder eines ophthalmologisch unbedenklichen Salzes davon zur gleichzeitigen, separaten oder aufeinanderfolgenden Verabreichung umfaßt.

11. Benutzung nach Anspruch 10, wobei die Konzentration an β-Adrenorezeptorenblocker 0,1 bis 0,5 Gew.% beträgt.

12. Benutzung nach Anspruch 11, wobei der β-Adrenorezeptorblocker Timololmaleat ist.

13. Benutzung nach Anspruch 11 oder 12, die außerdem 0,005 bis 2,0 Gew.% eines Prostaglandins, Prostaglandinderivats oder eines ophthalmologisch unbedenklichen Salzes davon zur gleichzeitigen, separaten oder aufeinanderfolgenden Verabreichung umfaßt.

14. Benutzung nach Anspruch 13, wobei die Konzentration an Prostaglandin oder Prostaglandinderivat 0,1 bis 1 Gew.% beträgt.

15. Benutzung nach Anspruch 14, wobei das Prostaglandin oder Prostaglandinderivat ist:
11-Pivaloyl-prostaglandin-F2α-hydroxyethylester,
(+)-(Z)-Natrium-7-[1R,2R,3R,5S)-3,5-dihydroxy-2-[(E)-1-octenyl]cyclopentyl]-5-heptenoat-sesquihydrat,
[1α,2β,3α,5α]Methyl-5-cis-2-(phenylethylsulfonamidomethyl)-3,5-dihydroxycyclopentylheptenoat,
(+-)-5-[6-(1-Hydroxy)hexyl)-1,3-benzodioxol-5-yl]-pentanol,
15-Pivaloyl-PGFα,
7-[3α,5α-Dihydroxy-2-(3a-hydroxy-5-1E-pentenyl)cyclopentyl]-5Z-heptensäure,
Isopropyl(Z)-7-[(1R,2R,3R,5S)-3,5-dihydroxy-2-[(3R)-3-hydroxy-5-phenylpentyl]cyclopentyl]-5-heptenoat oder
13,14-Dihydro-15-keto-20-ethyl-PGF2α-isopropylestertrimethylphenol-1-acetat.

16. Benutzung nach Anspruch 15, wobei das Prostaglandin oder Prostaglandinderivat ist Isopropyl-(Z)-7-[(1R,2R,3R,5S)-3,5-dihydroxy-2-[(3R)-3-hydroxy-5-phenylpentyl] cyclopentyl] -5-heptenoat, (+) - (Z) - Natrium-7-[1R,2R,3R,5S)-3,5-dihydroxy-2-[(E)-1-octenyl]cyclopentyl]-5-heptenoat-sesquihydrat oder 13,14-Dihydro-15-keto-20-ethyl-PGF2α-isopropylestertrimethylphenol-1-acetat ist.

## Revendications

1. Utilisation d'un inhibiteur de l'anhydrase carbonique pour fabriquer un médicament pouvant être appliqué sur l'oeil afin d'augmenter la tension de l'oxygène dans le nerf optique et la rétine, à l'exception du traitement du glaucome.

2. Utilisation selon la revendication 1, dans laquelle le médicament comprend une solution hypotonique comprenant de 0, 1 à 2 % (V/V) de gomme de xanthane et de 0,5 à 5 % (V/V) d'un inhibiteur de l'anhydrase carbonique.

3. Utilisation selon la revendication 2, dans laquelle l'inhibiteur de l'anhydrase carbonique est un composé de formule développée (I) : 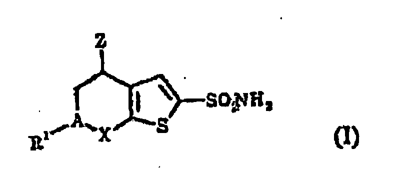 un diastéréomère individuel, un énantiomère individuel, ou un mélange de ceux-ci, ou un sel ophtalmologiquement acceptable de ceux-ci, dans laquelle :
A est du carbone ou de l'azote, de préférence du carbone ;
Z est -NHR ou -OR, de préférence -NHR ;
R est un alkyle en C₁ à C₆, à chaîne linéaire ou ramifiée, de préférence un alkyle en C₂ à C₄ tel que l'éthyle, le propyle ou l'isobutyle ;
R¹est
(a) de l'hydrogène,
(b) un alkyle en C₁à C₃, de préférence du méthyle, de l'éthyle ou du n-propyle, ou
(c) un alcoxyalkyle en C₁ à C₄, de préférence du 3-méthoxypropyle ou de l'éthoxyméthyle ; et
x est -S (O)₂- ou -C(O)-, de préférence -S(O)₂-.

4. Utilisation selon la revendication 3, dans laquelle A est du carbone ; et dans laquelle R est -CH₂CH₃ et R¹ est -CH₃ ; ou R est -CH₂CH₂CH₃ et R¹ est -CH₂CH₂CH₂OCH₃ ; ou R est -CH₂CH₃ et R¹ est -CH₂CH₂CH₃ ; ou R est -CH₂CH₂(CH₃)₂ et R¹ est de l' hydrogêne ; ou R est - CH₂CH₃ et R¹ est -CH₂OCH₂CH₃ ; et les carbones 4 et 6 de l'inhibiteur topique de l'anhydrase carbonique ont tous deux une configuration stéréochimique absolue en *S.*

5. Utilisation selon la revendication 2 ou 4, dans laquelle l'inhibiteur de l'anhydrase carbonique est choisi dans le groupe comprenant le dorzolamide, le brinzolamide, l'acétazolamide, le métazolamide et similaires.

6. Utilisation selon la revendication 5, dans laquelle l'inhibiteur de l'anhydrase carbonique est le dorzolamide.

7. Utilisation selon la revendication 6, dans lequel le dorzolamide est le chlorhydrate de (S,S)-(-)-5,6-dihydro-4-éthylamino-6-méthyl-4*H*-thiéno [2,3-b]thiopyran-2-sulfonamide-7,7-dioxyde.

8. Utilisation selon l'une quelconque des revendications 2 à 7, dans laquelle la concentration d'inhibiteur de l'anhydrase carbonique est comprise entre 0,5 et 2,0 % (V/V) et la concentration de gomme de xanthane est comprise entre 0,4 et 0,7 % (V/V).

9. Utilisation selon l'une quelconque des revendications 2 à 8, dans laquelle la composition est une solution hypotonique avec un abaissement du point de congélation entre -0,28 °C et -0,4 °C et l'hypotonicité de la solution est comprise entre 150 et 215 mOs/kg.

10. Utilisation selon l'une quelconque des revendications 1 à 9, dans laquelle le médicament comprend en plus de 0,01 à 1 % (V/V) d'un agent bloquant le récepteur β-adrénergique, ou d'un sel ophtalmologiquement acceptable de celui-ci, pour une administration simultanée, séparée ou séquentielle.

11. Utilisation selon la revendication 10, dans laquelle la concentration de l'agent bloquant le récepteur β-adrénergique est comprise entre 0,1 et 0,5 % (V/V).

12. utilisation selon la revendication 11, dans laquelle l'agent bloquant le récepteur β-adrénergique est le maléate de timolol.

13. Utilisation selon la revendication 11 ou 12, qui comprend en plus de 0,005 à 2,0 % (V/V) de prostaglandine, d'un dérivé de la prostaglandine, ou d'un sel ophtalmologiquement acceptable de celles-ci, pour une administration simultanée, séparée ou séquentielle.

14. Utilisation selon la revendication 13, dans laquelle la concentration de prostaglandine ou d'un dérivé de la prostaglandine est comprise entre 0,1 et 1 % (V/V).

15. Utilisation selon la revendication 14, dans lequel la prostaglandine ou un dérivé de la prostaglandine est :
l'ester hydroxyéthylique de prostaglandine F2α de 11-pivaloyle,
le sesquihydrate de (+)-(Z)-sodium-7-[1R, 2R, 3R, 5S]-3,5-dihydroxy-2- [(E) -1-octényl] cyclopentyl] -5-heptènoate,
l'heptanoate de [1α,2β,3α,5α]méthyl-5-cis-2-(phényléthylsulfonamidométhyl)-3,5-dihydroxycyclopentyle,
le (+-)-5-[6-(1-hydroxy)hexyl)-1,3-benzodioxol-5-yl]-pentanol,
la PGFα de 15-pivaloyle,
l'acide 7-[3α,5-dihydroxy-2-(3a-hydroxy-5-1E-pentényl)cyclopentyl]-5Z-hepténoïque,
le (Z)-7-[(1R,2R,3R,5S)-3,5-dihydroxy-2-[(3R)-3-hydroxy-5-phénylpentyl]cyclopentyl]-5-heptènoate d'isopropyle ou
le triméthylphénol-1-acétate de l'ester 13,14-dihydro-15-céto-20-éthyl-PGF2α isopropylique

16. Utilisation selon la revendication 15, dans laquelle la prostaglandine ou un dérivé de la prostaglandine est le (Z) -7- [(1R, 2R, 3R, 5S) -3, 5-dihydroxy-2- [(3R)-3-hydroxy-5-phénylpentyl]cyclopentyl]-5-heptènoate d'isopropyle, le sesquihydrate de (+)-(Z)-sodium-7-[1R,2R,3R,5S)-3,5-dihydroxy-2-[(E)-1-octé-nyl]cyclopentyl]-5-heptènoate, ou le triméthylphénol-1-acétate de l'ester 13,14-dihydro-15-céto-20-éthyl-PGF2α isopropylique.
